(19) Europäisches Patentamt
European Patent Office
Office européen des brevets

(11) Numéro de publication: **0 233 800 B1**

(12) # FASCICULE DE BREVET EUROPÉEN

(45) Date de publication du fascicule du brevet: **28.02.90**

(21) Numéro de dépôt: **87400073.0**

(22) Date de dépôt: **14.01.87**

(51) Int. Cl.[5]: **C 07 D 471/04**, A 61 K 31/435 // (C07D471/04, 221:00, 221:00)

(54) **Dérivés d'imidazo 1,2-a quinoléines, leur préparation et leur application en thérapeutique.**

(30) Priorité: **22.01.86 FR 8600834**

(43) Date de publication de la demande:
**26.08.87 Bulletin 87/35**

(45) Mention de la délivrance du brevet:
**28.02.90 Bulletin 90/09**

(84) Etats contractants désignés:
**AT BE CH DE ES FR GB GR IT LI LU NL SE**

(56) Documents cité:
**EP-A-0 050 563**
**EP-A-0 172 097**

(73) Titulaire: **SYNTHELABO**
**58 rue de la Glacière**
**F-75013 Paris (FR)**

(72) Inventeur: **George, Pascal**
**39, rue Henri de Vilmorin**
**F-94400 Vitry S/Seine (FR)**
Inventeur: **de Peretti, Danièle**
**14, rue Ampère**
**F-92160 Antony (FR)**

(74) Mandataire: **Ludwig, Jacques**
**SYNTHELABO Service Brevets 58, rue de la Glacière**
**F-75621 Paris Cedex 13 (FR)**

EP 0 233 800 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe dipposition (Art. 99(1) Convention sur le brevet européen).

LIBERGRAF, STOCKHOLM 1990

## Description

La présente invention a pour objet des dérivés d'imidazo [1,2-a] quinoléines de formule générale (I), figurant dans le schéma de la page suivante, formule dans laquelle

- X représente l'hydrogène, un halogène ou un groupe $(C_{1-4})$ alkyle, $(C_{1-4})$alcoxy, $(C_{1-4})$alkylthio, méthylsulfonyle, amino, $(C_{1-4})$alkylamino, di-$(C_{1-4})$alkylamino, nitro ou trifluoroalkyle,
- Y représente l'hydrogène, un halogène ou un groupe méthyle en position 6, 7 ou 8 et
- R1 et R2 pris séparément, représentent chacun l'hydrogène ou un groupe $(C_{1-6})$alkyle, ou bien
- R1 et R2 forment ensemble une chaîne tétraméthylène, pentaméthylène, méthyl-3 aza-3 pentaméthylène ou oxa-3 pentaméthylène.

Les sels d'addition que peuvent former ces composés avec des acides font également partie de l'invention.

Les composés préférés de l'invention sont ceux pour lesquels

- X représente un atome de chlore, le groupe méthyle ou le groupe méthylthio, en position 4,
- Y représente un atome d'hydrogène, R1 et R2 représentent chacun, indépendamment l'un et l'autre, un atome d'hydrogène ou le groupe méthyle.

**Schéma**

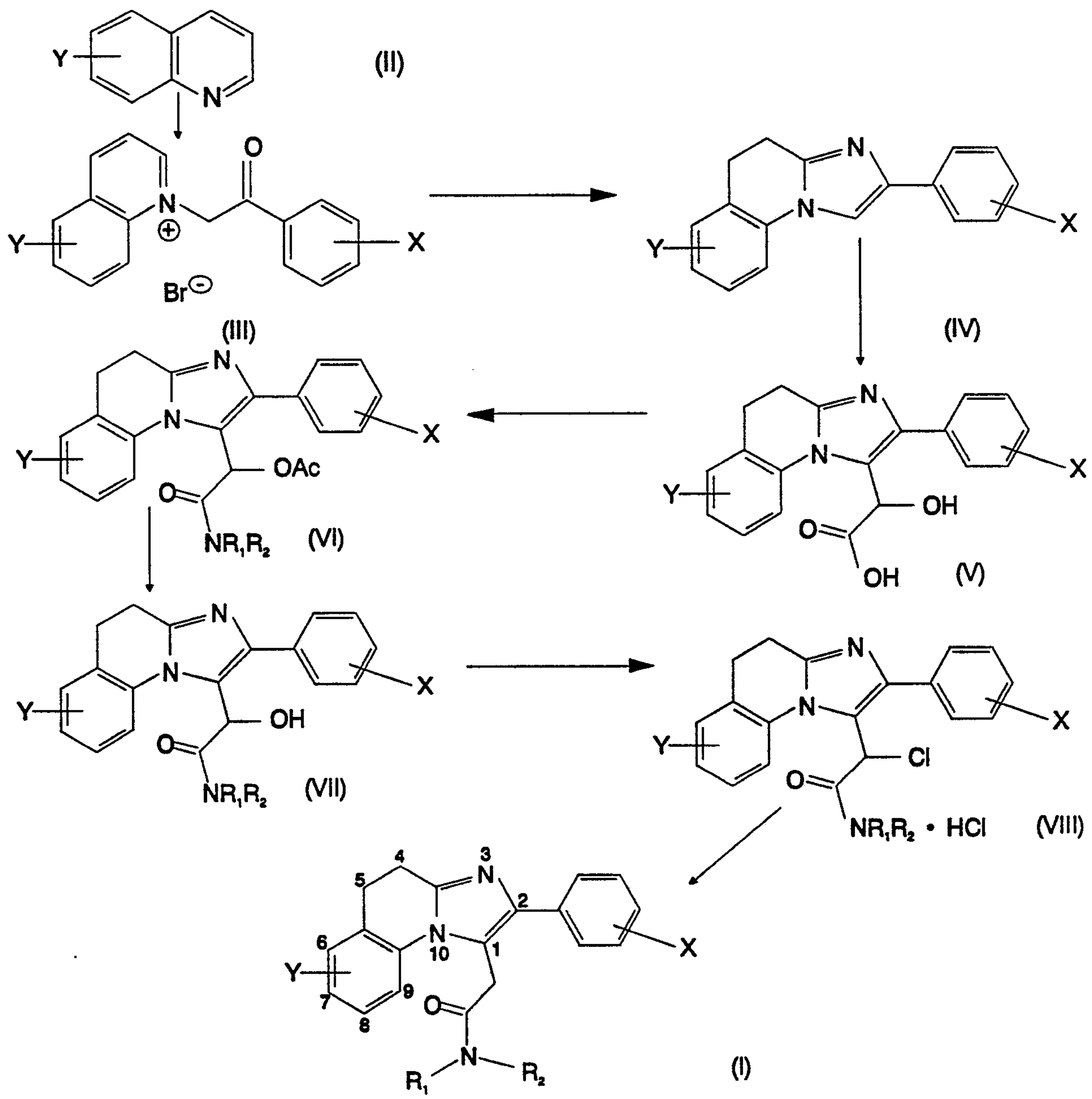

(II)
(III)
(IV)
(V)
(VI)
(VII)
(VIII)
(I)

Les composés de l'invention peuvent être préparés selon le schéma de la page suivante.
On soumet d'abord la quinoléine de formule (II) à l'action d'une α-bromo-acétophénone portant le substituant X défini ci-dessus. La réaction a lieu à chaud dans un solvant tel que le chlorure de méthylène ou le dichloro-1,2 éthane. On obtient un composé ionique de formule (III) que l'on cyclise en présence d'acétate d'ammonium dans un solvant organique acide tel que l'acide acétique ou propionique, à la température de 90°C, pour obtenir le composé de formule (IV). On fait réagir le composé obtenu (IV) avec de l'acide glyoxylique dans un solvant tel que l'acide acétique à 80°C. On acétyle l'hydroxy-acide obtenu (V) à l'aide d'anhydride acétique en présence de pyridine puis on le transforme en α-acétoxy-acétamide (VI) via l'imidazolide préparé in situ.

On désacétyle le composé (VI) en α-hydroxy-acétamide (VII) par traitement avec du carbonate de potassium dans de l'éthanol.

On fait réagir le composé (VII) avec du chlorure de sulfonyle $SOCl_2$ dans un solvant chloré tel que le dichlorométhane pour obtenir le composé chloré (VIII) que l'on réduit à l'aide de Rongalite® dans du chlorure de méthylène en composé (I).

Les exemples suivants illustrent l'invention.

Les microanalyses et les spectres IR et RMN confirment la structure des composés (I).

**Exemple 1**

N-méthyl (méthyl-4 phényl)-2 dihydro-4,5 imidazo [1,2-a] quinoléine-1-acétamide.

1.1. Bromure de [(méthyl-4 phényl)-2 oxo-2 éthyl]-1 quinoléinium.

On dissout 110 g (0,516 mole) d'α-bromo-para-méthyl-acétophénone et 61 ml (0,516 mole) de quinoléine dans 500 ml de chlorure de méthylène.
On chauffe la solution à la température du reflux pendant 1 heure puis on la dilue avec 300 ml d'éther et on la refroidit.
On obtient, après filtration et séchage du précipité, un solide jaune.
F = 220 – 221°C.

1.2. (méthyl-4 phényl)-2 dihydro-4,5 imidazo [1,2-a] quinoléine.

On mélange, dans 50 ml d'acide acétique, 17,1 g (0,05 mole) de sel quaternaire obtenu en 1.1 et 25 g d'acétate d'ammonium. On chauffe cette suspension pendant 3 heures à 90°C, puis la refroidit et la dilue au moyen de 200 ml d'eau. On filtre le précipité brun formé et le reprend entre l'eau et le chlorure de méthylène. On traite ce mélange biphasique avec un excès de NaOH 1N jusqu'à pH ≥ 8; on décante la phase organique, la sèche sur $Na_2SO_4$, la filtre et concentre le filtrat sous pression réduite. On fait recristalliser le résidu d'évaporation dans du pentane.
On obtient ainsi la (méthyl-4 phényl)-2 dihydro-4,5 imidazo [1,2-a] quinoléine (IV).
F = 91 – 92°C (décomposition).

1.3. Acide α-hydroxy (méthyl-4 phényl)-2 dihydro-4,5 imidazo [1,2-a] quinoléine-1-acétique

On chauffe, 6 heures à 80°C, un mélange de 29 g (0,112 mole) de dihydro-4,5 imidazo [1,2-a] quinoléine obtenue en 1.2., de 16,3 g (0,225 mole) d'acide glyoxylique et de 550 ml d'acide acétique. On concentre la solution sous pression réduite et reprend le résidu d'évaporation par de l'eau. L'acide cristallise, on le filtre, le lave à l'eau puis au THF et à l'éther. On le sèche sous vide. On obtient l'α-hydroxy-acide (V).
F = 178 – 181°C (décomposition).

1.4. α-hydroxy N-méthyl (méthyl-4 phényl)-2 dihydro-4,5 imidazo [1,2-a] quinoléine-1-acétamide

1.4.1. On dissout 16,5 g (0,049 mole) d'α-hydroxy-acide dans 300 ml d'un mélange 50/50 de pyridine et d'anhydride acétique. On agite ce mélange une nuit à la température ambiante puis on le concentre sous pression réduite. Le résidu d'évaporation cristallise par traitement à l'éther. On obtient un produit que l'on utilise directement pour l'étape suivante.

1.4.2. On fait réagir 16,5 g (0,045 mole) d'α-acétyloxyacide ainsi obtenu et 9,5 g (0,058 mole) de carbonyldiimidazole dans 200 ml de THF sec. En fin de dégagement gazeux, on porte la solution 1 heure à 50°C puis la refroidit et la traite par un excès de méthylamine gazeuse sèche. On concentre le mélange réactionnel sous pression réduite, reprend le résidu entre l'eau et le chlorure de méthylène et le traite au $K_2CO_3$. On agite ce mélange 3 heures à la température ambiante puis le décante. On sèche la phase organique au $Na_2SO_4$, la filtre et évapore le filtrat sous pression réduite. On purifie le mélange obtenu par chromatographie sur silice et le fait cristalliser dans de l'éther. On obtient l'α-acétyloxyacétamide (VI) que l'on désacétyle directement sans autre purification.

1.4.3. On traite l'α-acétyloxy-acétamide (VI) par 25 g de $K_2CO_3$ dans 100 ml de méthanol aqueux à 50 %. On maintient l'agitation toute la nuit. On concentre la solution, reprend le résidu solide à l'eau, le filtre et le lave à l'eau jusqu'à pH neutre des eaux de lavage, puis le lave à l'éther et le sèche. On obtient l'α-hydroxy-acétamide (VII).

F = 209 – 211°C.

1.5. N-méthyl (méthyl-4 phényl)-2 dihydro-4,5 imidazo [1,2-a] quinoléine-1-acétamide

On traite 4,0 g (0,0115 mole) d'α-hydroxy-acétamide (VII) par 25 ml de $SOCl_2$ dans 125 ml de chlorure de méthylène à la température ambiante pendant une nuit. On évapore les résidus volatiles. On obtient le chlorhydrate de l'α-chloro-acétamide (VIII). On dissout 100 g (0,0115 mole) de ce composé dans 150 ml de $CH_2Cl_2$. On traite cette solution par 5,3 g (0,0345 mole) de Rongalite® à la température ambiante pendant 24 heures. En fin de réaction, on filtre le mélange, concentre le filtrat sous pression réduite et traite le résidu d'évaporation avec $NaHCO_3$ aqueux. Après plusieurs lavages à l'eau, on filtre le résidu solide et le sèche. On purifie le mélange brut par chromatographie puis le fait recristalliser dans de l'acétonitrile. On obtient l'amide (I).
F = 226 – 8°C.

**Exemple 2**

N,N-diméthyl (méthyl-4 phényl)-2 dihydro-4,5 imidazo [1,2-a] quinoléine-1-acétamide.

2.1. α-hydroxy N,N-diméthyl (méthyl-4 phényl)-2 dihydro-4,5 imidazo [1,2-a] quinoléine-1-acétamide

2.1.1. On fait réagir 16,5 g (0,049 mole) d'α-acétyloxyacide obtenu sous 1.4.1. avec 9,5 g (0,0585 mole) de carbonyldiimidazole dans 200 ml de THF sec. En fin de dégagement gazeux, on porte la solution à 50°C pendant 1 heure puis la refroidit et la traite par un excès de diméthylamine gazeuse sèche. On agite le mélange plusieurs heures puis le concentre sous pression réduite. On traite le résidu d'évaporation par $NaHCO_3$ aqueux et extrait l'α-acétyloxy-amide au $CH_2Cl_2$. On le purifie par chromatographie sur silice. On obtient une huile.

2.1.2. On traite l'huile obtenue précédemment par 45 g de $K_2CO_3$ dissous dans 100 ml de $CH_3OH$ à la température ambiante pendant 40 heures. Après évaporation de l'eau et du méthanol, on reprend le résidu entre l'eau et le chlorure de méthylène. On décante la phase organique, la sèche au $Na_2SO_4$ et la filtre. On concentre le filtrat sous pression réduite. On fait cristalliser l'huile résiduelle dans de l'éther. On obtient l'α-hydroxy-amide (VII).
F = 169 – 70°C.

2.2.. N,N-diméthyl (méthyl-4 phényl)-2 dihydro-4,5 imidazo [1,2-a] quinoléine-1-acétamide

On traite 4 g (0,0115 mole) d'α-hydroxy-acétamide (VII) par 25 ml de $SOCl_2$ dans 125 ml de $CH_2Cl_2$ à la température ambiante pendant une nuit. Après évaporation du solvant et de l'excès de $SOCl_2$, on obtient le chlorhydrate de l'α-chloro-acétamide brut que l'on dissout dans 150 ml de $CH_2Cl_2$. On traite cette solution par 5,3 g (0,0345 mole) de Rongalite® à la température ambiante pendant 24 heures. En fin de réaction, on filtre la suspension, concentre le filtrat sous pression réduite et traite le résidu d'évaporation au $NaHCO_3$ aqueux. On extrait le solide insoluble par $CH_2Cl_2$ et le purifie par chromatographie (silice). On le fait recristalliser dans de l'acétate d'éthyle. On obtient l'acétamide (I).
F = 206 – 207°C.

**Tableau**

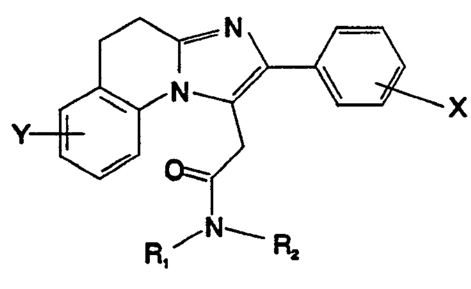

(I)

| Composé | Y | X | $R_1$ | $R_2$ | F(°C) |
|---|---|---|---|---|---|
| 1 | H | 4-$CH_3$ | H | $CH_3$ | 226 – 8 |
| 2 | H | 4-$CH_3$ | $CH_3$ | $CH_3$ | 206 – 7 |
| 3 | H | 4-Cl | H | $CH_3$ | 236 – 7 |
| 4 | H | 4-Cl | $CH_3$ | $CH_3$ | 193 – 4 |
| 5 | H | 4-$SCH_3$ | H | $CH_3$ | 245 – 7 |

Les composés de l'invention ont fait l'objet d'essais pharmacologiques qui ont mis en évidence leur intérêt comme substances à activités thérapeutiques.

Antagonisme vis-à-vis des convulsions cloniques induites par le Cardiazol® chez la souris.

L'essai est inspiré du protocole décrit par Goodman et al., J. Pharm. Exp. Ther., *108*, 168-176. Les souris reçoivent les produits à tester, ou le solvant seul, 30 minutes (voie i.p.) avant l'injection de 35 mg/kg de Cardiazol® par voie intraveineuse. Les animaux sont ensuite observés pendant une heure et, pour chaque lot, le pourcentage de souris présentant des convulsions cloniques est noté (100 % de convulsions cloniques et 10 à 20 % de convulsions toniques chez les animaux de contrôle).

Pour chaque dose, on calcule le pourcentage de protection par rapport aux animaux de contrôle, ce qui permet de déterminer graphiquement la $DA_{50}$, dose qui protège 50 % des animaux vis-à-vis des effets convulsivants du Cardiazol®. Les $DA_{50}$ des composés de l'invention se situent entre 0,1 et 30 mg/kg pour la voie intrapéritonéale et entre 0,1 et 30 mg/kg pour la voie orale.

**Test "d'enfouissement" chez la souris ("Burying test")**

Ce test est inspiré de la méthode décrite par Pinel J.P.J., Treit D., Ladak F. et MacLennan A.J. dans Animal learning and behavior, *8*, 447-451, (1980).

La présence de corps étrangers dans l'environnement habituel d'un animal constitue une situation aversive à laquelle l'animal réagit en enfouissant l'objet de l'agression (billes de verre) dans la sciure de sa cage.

Les anxiolytiques ont pour effet de diminuer l'appréhension causée par la présence étrangère: les animaux enfouissent moins. On compte alors le nombre de billes restées non enfouies.

Les produits à étudier sont administrés à des souris mâles de souche CDI (Charles River) 30 minutes (voie intrapéritonéale) ou 60 minutes (voie orale) avant que ces dernières soient placées dans des cages contenant 25 billes de verre. Au bout de 30 minutes on compte le nombre de billes restées non enfouies. Un pourcentage est calculé entre les animaux traités et les animaux témoins.

On détermine ainsi la $DA_{50}$, dose active 50 %, qui est la dose de composé (en mg/kg) diminuant de moitié le nombre de billes enfouies, en comparaison avec les animaux témoins. Les $DA_{50}$ des composés de l'invention se situent entre 0,3 et 30 mg/kg par voie intrapéritonéale.

**Test de conflit de le boisson chez le rat**

Ce test décrit par Vogel J.R., Beer B. et Clody D.E. dans Psychopharmacologia, *21*, 1-7, (1971). Des rats mâles Wistar (IFFA Credo) sont utilisés. L'eau de boisson leur est retirée 24 h avant le test. Le jour du test, 30 minutes après traitement par voie intrapéritonéale avec les composés de l'invention, chaque rat est placé dans une cage en matière plastique transparente (24 x 20 x 21 cm) à plancher grillagé électrifiable. De l'eau de boisson est distribuée par l'intermédiaire d'une pipette sortant de 2 cm d'une paroi de la cage et placée à 3 cm au dessus du plancher de la cage.

Après une exploration de 10 à 90 secondes, l'animal trouve la pipette et commence à boire. Après avoir donné 20 coups de langue (enregistrés par un anxiomètre OMNITECH), le rat reçoit, au niveau de la langue un choc électrique de 0,07 mA (délivré par l'anxiomètre) qui s'arrête lorsque le rat quitte la pipette. Une session de 3 minutes commence après un premier choc, l'animal continuant de recevoir un choc tous les 20 coups de langue jusqu'à ce qu'il s'arrête ou jusqu'à la fin de la session.

Dans ces conditions expérimentales, les animaux de contrôle acceptent, en moyenne, 3 à 6 chocs. Le nombre de chocs obtenus avec les animaux traités est noté, et on compare ce nombre avec celui des animaux témoins par un test de Dunett.

On détermine ainsi la DEM, dose efficace minimale, qui est la première dose augmentant de façon significative le nombre de chocs acceptés par un animel, par rapport aux animaux témoins.

Les DEM se situent entre 3 et 100 mg/kg par voie intrapéritonéale.

Action sur l'électrocorticogramme du rat curarisé ventilé.

L'activité sédative ou hypnotique des composés a été déterminée par l'observation de leur action sur l'électrocorticogramme du rat selon la méthode décrite par H. Depoortere, Rev. E.E.G. Neurophysiol., *10*, 3, 207-214 (1980) et par H. Depoortere et M. Decobert, J. Pharmacol. (Paris), *14*, 2, 195-265 (1983).

Les produits à étudier ont été administrés par voie intrapéritonéale aux doses croissantes de 1 à 30 mg/kg. Ils induisent des tracés de sommeil à partir de doses allant de 3 à 100 mg/kg.

**Test de Koster**

L'activité analgésique a été montrée dans le test de Koster et al. ("writhing test" à l'acide acétique chez la souris), Fed. Proc., *18*, 412, 1959.

On administre par voie orale, aux souris à jeun, le composé à tester en solution dans du Tween 80 à 1 %, à raison de 0,2 ml par 20 g de poids corporel; au bout de 30 mn on administre l'acide acétique (en solution à 0,6 % dans un mélange carboxyméthyl-cellulose et Tween 80, à raison de 10 ml par kg de poids corporel) par voie intrapéritonéale. On note le nombre total de contorsions pendant 15 mn.

On détermine le pourcentage de protection par rapport à un lot témoin et on calcule la DA 50 par voie graphique (dose qui protège 50 % des animaux).

La DA 50 des composés de l'invention va de 5 à 50 mg/kg p.o.

**Test anti-ulcère de stress**

La technique utilisée est celle de Senay et Levine, Proc. Soc. Exp. Biol. 1967, *124*, 1221-1223, Peptic Ulcers, sur des rats Wistar femelles pesant 180 – 210 g, tenus à jeun depuis 20 heures, répartis en blocs randomisés.

Les animaux sont mis en contention dans des boîtes cylindriques de 20 cm x 5 cm et placés dans une chambre froide dont la température est maintenue entre 2 et 4°C.

Les composés à étudier sont administrés par voie orale à raison de 10, 30 et 100 mg/kg immédiatement avant la mise en contention, les rats témoins recevant seulement le placébo. 2 heures plus tard, les animaux sont sacrifiés par inhalation de chloroforme.

Les estomacs sont prélevés et le degré d'ulcération est noté.

Les composés de l'invention diminuent significativement les ulcères de stress.

Les résultats de ces différents tests montrent que les composés de l'invention possèdent des propriétés anxiolytiques, inductrices de sommeil, hypnotiques, anticonvulsivantes, analgésiques et antiulcères; les composés de l'invention sont utiles pour le traitement des états d'anxiété, des troubles du sommeil et autres affections neurologiques et psychiatriques, pour le traitement des troubles de la vigilance, en particulier pour lutter contre les troubles du comportement imputables à des dommages vasculaires cérébraux et à la sclérose cérébrale en gériatrie, pour le traitement des absences dues à des traumatismes crâniens et pour le traitement des encéphalopathies métaboliques ainsi que pour le traitement des algies, de la douleur et des ulcères.

Les composés de l'invention peuvent être présentés sous toute forme appropriée pour l'administration par voie orale ou parentérale, par exemple sous forme de comprimés, de dragées, de gélules, de solutions buvables ou injectables etc, en association avec tout excipient approprié.

La posologie quotidienne peut aller de 1 à 100 mg.

**Revendications** pour les Etats Contractants: BE, CH, DE, FR, GB, IT, LI, LU, NL, SE

1. Dérivés d'imidazo [1,2-a] quinoléines répondant à la formule

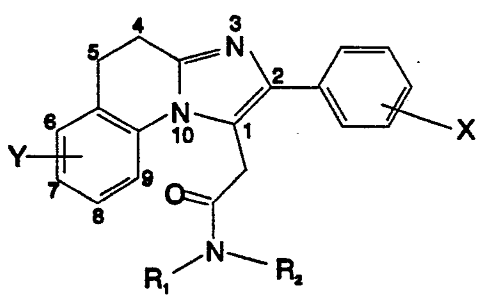

(I) .

dans laquelle

X représente l'hydrogène, un halogène ou un groupe $(C_{1-4})$alkyle, $(C_{1-4})$alcoxy $(C_{1-4})$alkylthio, méthylsulfonyle, amino, $(C_{1-4})$alkylamino, di-$(C_{1-4})$alkylamino, nitro ou trifluoroalkyle,

Y représente l'hydrogène, un halogène ou un groupe méthyle en position 6, 7 ou 8 et

R1 et R2 pris séparément, représentent chacun l'hydrogène ou un groupe $(C_{1-6})$alkyle, ou bien

R1 et R2 forment ensemble une chaîne tétraméthylène, pentaméthylène, méthyl-3 aza-3 pentaméthylène ou oxa-3 pentaméthylène,

ainsi que leurs sels d'addition acceptables en pharmacologie.

2. Dérivés selon la revendication 1, caractérisés par le fait que X représente un atome de chlore, le groupe méthyle ou le groupe méthylthio, en position 4, Y représente un atome d'hydrogène, et R1 et R2 représentent chacun, indépendamment l'un et l'autre, un atome d'hydrogène ou le groupe méthyle.

3. Procédé de préparation de composés selon la revendication 1, procédé caractérisé en ce que l'on soumet d'abord la quinoléine de formule (II)

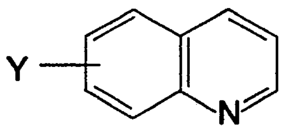

(II)

a l'action d'une α-bromo-acétophénone portant le substituant X défini ci-dessus, à chaud dans un solvant tel que le chlorure de méthylène ou le dichloro-1,2 éthane; on obtient un composé ionique de formule (III)

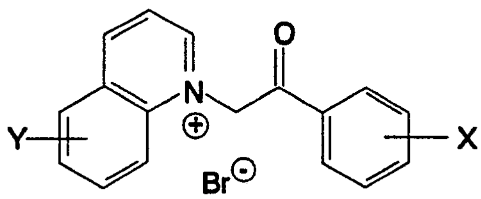

(III)

que l'on cyclise en présence d'acétate d'ammonium dans un solvant organique acide tel que l'acide acétique ou propionique, à la température de 90°C, pour obtenir le composé de formule (IV)

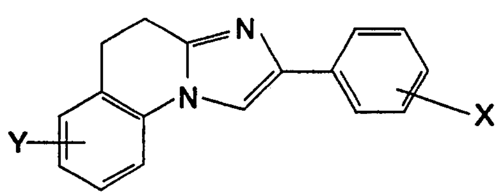

(IV)

on fait réagir le composé obtenu (IV) avec de l'acide glyoxylique dans un solvant tel que l'acide acétique à 80°C; on acétyle l'α-hydroxy-acide obtenu (V)

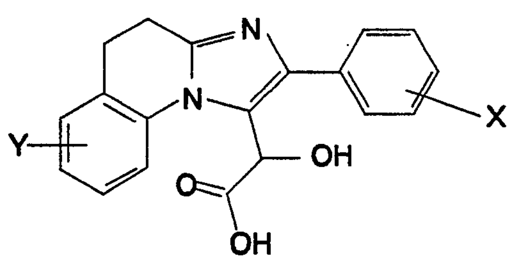

(V)

à l'aide d'anhydride acétique en présence de pyridine puis on le transforme en α-acétyloxy-acétamide

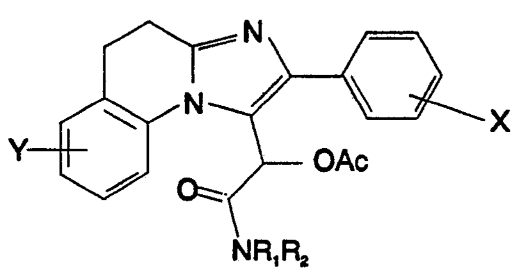

(VI)

via l'imidazolide préparé in situ; on désacétyle le composé (VI) en α-hydroxy-acétamide (VII)

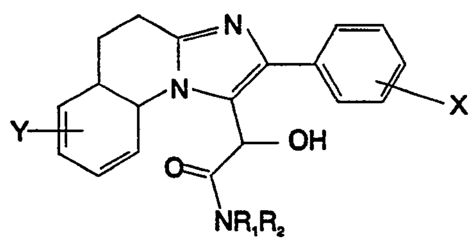

(VII)

par traitement avec du carbonate de potassium dans de l'éthanol; on fait réagir le composé (VII) avec du chlorure de sulfonyle $SOCl_2$ dans un solvant chloré tel que le dichlorométhane pour obtenir le composé chloré (VIII)

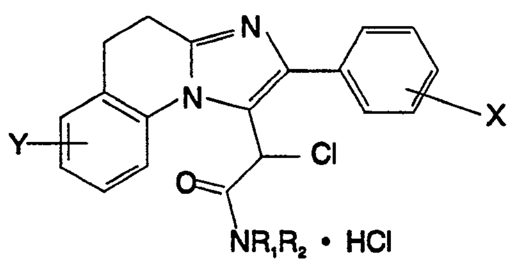

(VIII)

que l'on réduit a l'aide de Rongalite® dans du chlorure de méthylène en composé (I).

4. Médicament, caractérisé en ce qu'il est constitué par un composé selon l'une quelconque revendications 1 et 2.

5. Composition pharmaceutique, caractérisée en ce qu'elle contient un composé selon l'une quelconque revendications 1 et 2, en association avec un excipient approprié.

**Revendications** pour les Etats Contractants: AT, ES, GR

1. Procédé de préparation de dérivés d'imidazo [1,2-a] quinoléines répondant à la formule (I)

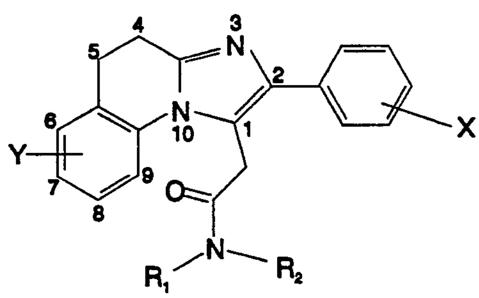

(I)

dans laquelle

| | |
|---|---|
| X | représente l'hydrogène, un halogène ou un groupe $(C_{1-4})$alkyle, $(C_{1-4})$alcoxy $(C_{1-4})$alkylthio, méthylsulfonyle, amino, $(C_{1-4})$alkylamino, di-$(C_{1-4})$alkylamino, nitro ou trifluoroalkyle, |
| Y | représente l'hydrogène, un halogène ou un groupe méthyle en position 6, 7 ou 8 et |
| R1 et R2 | pris séparément, représentent chacun l'hydrogène ou un groupe $(C_{1-6})$alkyle, ou bien |
| R1 et R2 | forment ensemble une chaîne tétraméthylène, pentaméthylène, méthyl-3 aza-3 pentaméthylène ou oxa-3 pentaméthylène, |

procédé caractérisé en ce que l'on soumet d'abord la quinoléine de formule (II)

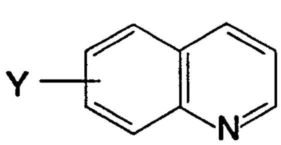

(II)

à l'action d'une α-bromo-acétophénone portant le substituant X défini ci-dessus, à chaud dans un solvant tel que le chlorure de méthylène ou le dichloro-1,2 éthane; on obtient un composé ionique de formule (III)

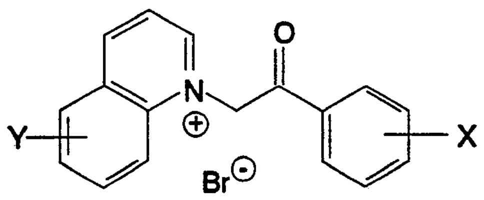

(III)

que l'on cyclise en présence d'acétate d'ammonium dans un solvant organique acide tel que l'acide acétique ou propionique, à la température de 90°C, pour obtenir le composé de formule (IV)

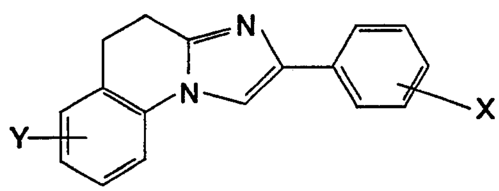

(IV)

on fait réagir le composé obtenu (IV) avec de l'acide glyoxylique dans un solvant tel que l'acide acétique à 80°C; on acétyle l'α-hydroxy-acide obtenu (V)

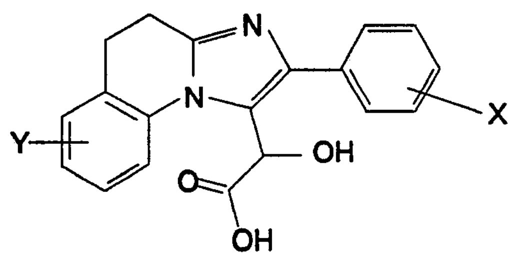

(V)

à l'aide d'anhydride acétique en présence de pyridine puis on le transforme en α-acétyloxy-acétamide

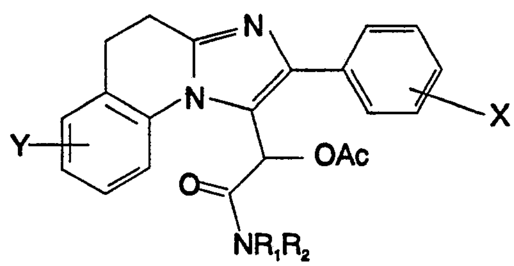

(VI)

via l'imidazolide préparé in situ; on désacétyle le composé (VI) en α-hydroxy-acétamide (VII)

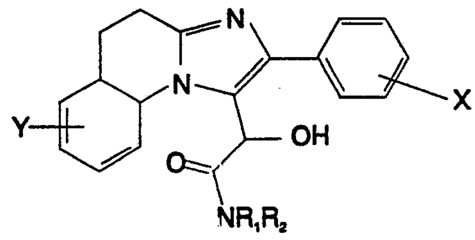

(VII)

par traitement avec du carbonate de potassium dans de l'éthanol; on fait réagir le composé (VII) avec du chlorure de sulfonyle $SOCl_2$ dans un solvant chloré tel que le dichlorométhane pour obtenir le composé chloré (VIII)

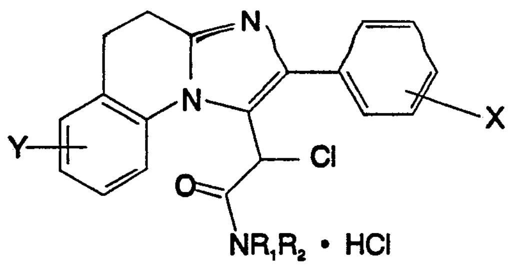

(VIII)

que l'on réduit à l'aide de Rongalite® dans du chlorure de méthylène en composé (I).

2. Procédé selon la revendication 1, caractérisés par le fait que X représente un atome de chlore, le groupe méthyle ou le groupe méthylthio, en position 4, Y représente un atome d'hydrogène, et R1 et R2 représentent chacun, indépendamment l'un et l'autre, un atome d'hydrogène ou le groupe méthyle.

**Patentansprüche** für die Vertragsstaaten: BE, CH, DE, FR, GB, IT, LI, LU, NL, SE

1. Imidazo[1,2-a]chinolin-Derivate gemäß der Formel (I)

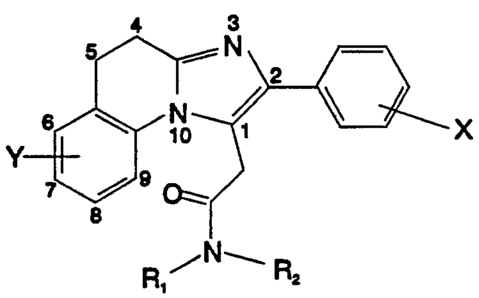

(I)

wobei

| | |
|---|---|
| X | Wasserstoff, Halogen oder eine der Gruppen $(C_{1-4})$-Alkyl, $(C_{1-4})$-Alkoxy, $(C_{1-4})$-Alkylthio, Methylsulfonyl, Amino, $(C_{1-4})$-Alkylamino, Di-$(C_{1-4})$-alkylamino, Nitro oder Trifluoralkyl bedeutet, |
| Y | Wasserstoff, Halogen oder eine Methylgruppe in der Stellung 6, 7 oder 8 bedeutet, und |
| R1 und R2 | einzeln jeweils Wasserstoff oder eine $(C_{1-6})$-Alkylgruppe bedeuten, oder jedoch |
| R1 und R2 | gemeinsam eine Tetramethylen-, Pentamethylen-, 3-Methyl-3-aza-pentamethylen- oder 3-Oxa-pentamethylenkette bilden, |

sowie ihre in der Pharmakologie verwendbaren Additionssalze.

2. Verbindungen nach Anspruch 1, dadurch gekennzeichnet, daß X ein Chloratom, eine Methylgruppe oder eine Methylthiogruppe in Stellung 4 bedeutet, Y ein Wasserstoffatom bedeutet und R1 und R2 einzeln jeweils unabhängig voneinander ein Wasserstoffatom oder eine Methylgruppe bedeuten.

3. Verfahren zur Herstellung von Verbindungen nach Anspruch 1, dadurch gekennzeichnet, daß zunächst ein Chinolin der Formel (II)

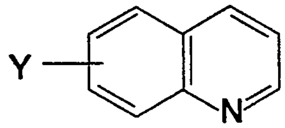

(II)

der Einwirkung eines α-Bromacetophenons, das den oben definierten Substituenten X trägt, in der Hitze in einem Lösungsmittel wie Methylenchlorid oder 1,2-Dichloräthan unterworfen wird;
man eine ionische Verbindung der Formel (III) erhält,

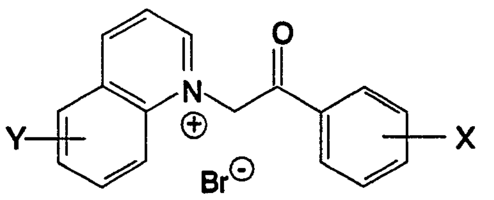

(III)

welche in Anwesenheit von Ammoniumacetat in einem organischen sauren Lösungsmittel wie Essigsäure oder Propionsäure bei einer Temperatur von 90°C zyklisiert wird, um die Verbindung der Formel (IV ) zu erhalten;

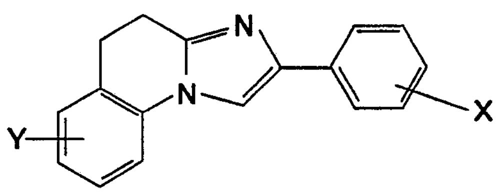

(IV)

die erhaltene Verbindung (IV) mit Glyoxylsäure in einem Lösungsmittel wie Essigsäure bei 80°C zur Reaktion gebracht wird;

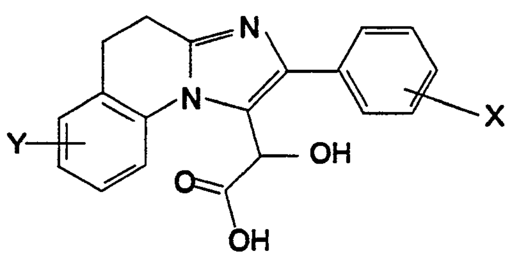

(V)

die erhaltene α-Hydroxysäure (V) mit Hilfe von Essigsäureanhydrid in Anwesenheit von Pyridin acetyliert und hierauf über das in situ hergestellte Imidazolid in das α-Acetyloxyacetamid übergeführt wird;

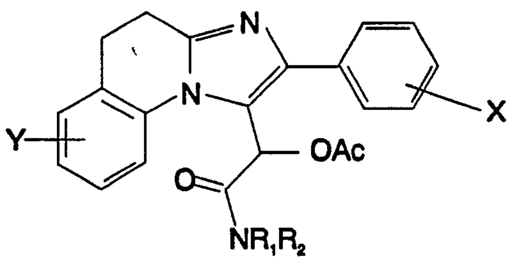

(VI)

die Verbindung (VI) durch Behandlung mit Kaliumcarbonat in Äthanol zum α-Hydroxyacetamid (VII) desacetyliert wird;

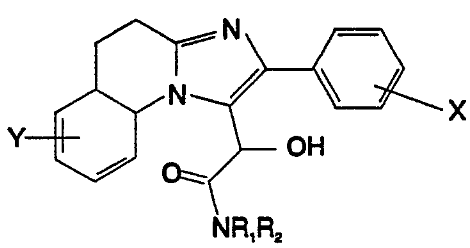

(VII)

die Verbindung (VII) mit Sulfonylchlorid $SOCl_2$ in einem chlorierten Lösungsmittel wie Dichlormethan zur Reaktion gebracht wird, um die chlorierte Verbindung (VIII) zu erhalten,

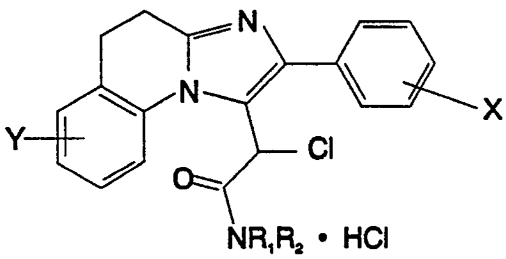

(VIII)

die mit Hilfe von Rongalite® in Methylenchlorid zur Verbindung (I) reduziert wird.

4. Arzneimittel, dadurch gekennzeichnet, daß es aus einer Verbindung nach einem der Ansprüche 1 und 2 besteht.

5. Pharmazeutische Zusammensetzung, dadurch gekennzeichnet, daß sie eine Verbindung nach einem der Ansprüche 1 und 2, gemeinsam mit einem geeigneten Exzipiens enthält.

**Patentansprüche** für die Vertragsstaaten: AT, ES, GR

1. Verfahren zur Herstellung von Imidazo[1,2-a]chinolin-Derivaten gemäß der Formel (I)

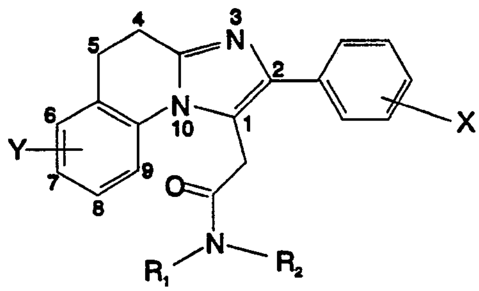

(I)

wobei

| | |
|---|---|
| X | Wasserstoff, Halogen oder eine der Gruppen $(C_{1-4})$-Alkyl, $(C_{1-4})$-Alkoxy, $(C_{1-4})$-Alkylthio, Methylsulfonyl, Amino, $(C_{1-4})$-Alkylamino, Di-$(C_{1-4})$-alkylamino, Nitro oder Trifluoralkyl bedeutet, |
| Y | Wasserstoff, Halogen oder eine Methylgruppe in der Stellung 6, 7 oder 8 bedeutet, und |
| R1 und R2 | einzeln jeweils Wasserstoff oder eine $(C_{1-6})$-Alkylgruppe bedeuten, oder jedoch |
| R1 und R2 | gemeinsam eine Tetramethylen-, Pentamethylen-, 3-Methyl-3-aza-pentamethylen- oder 3-Oxa-pentamethylenkette bilden, |

dadurch gekennzeichnet, daß zunächst ein Chinolin der Formel (II)

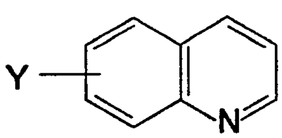

(II)

der Einwirkung eines α-Bromacetophenons, das den oben definierten Substituenten X trägt, in der Hitze in einem Lösungsmittel wie Methylenchlorid oder 1,2-Dichloräthan unterworfen wird;
man eine ionische Verbindung der Formel (III) erhält,

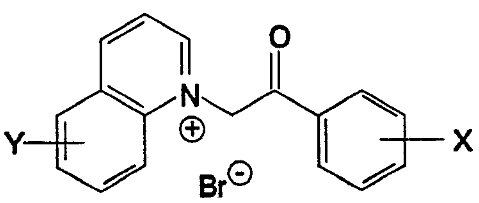

(III)

welche in Anwesenheit von Ammoniumacetat in einem organischen sauren Lösungsmittel wie Essigsäure oder Propionsäure bei einer Temperatur von 90°C zyklisiert wird, um die Verbindung der Formel (IV) zu erhalten;

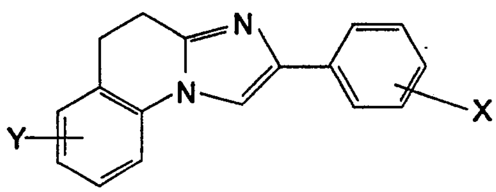

(IV)

die erhaltene Verbindung (IV) mit Glyoxylsäure in einem Lösungsmittel wie Essigsäure bei 80°C zur Reaktion gebracht wird;

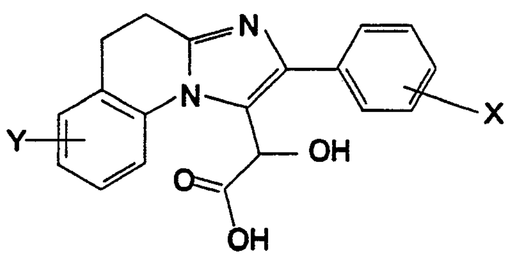

(V)

die erhaltene α-Hydroxysäure (V) mit Hilfe von Essigsäureanhydrid in Anwesenheit von Pyridin acetyliert und hierauf über das in situ hergestellte Imidazolid in das α-Acetyloxyacetamid übergeführt wird;

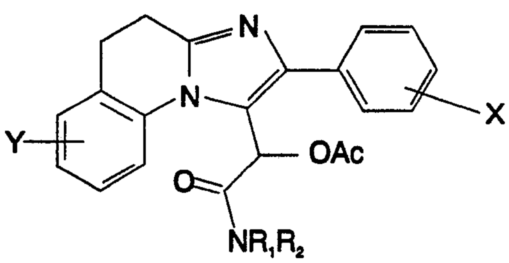

(VI)

die Verbindung (VI) durch Behandlung mit Kaliumcarbonat in Äthanol zum α-Hydroxyacetamid (VII) desacetyliert wird;

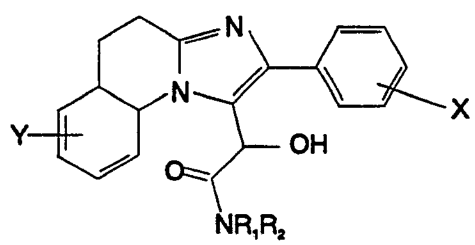

(VII)

die Verbindung (VII) mit Sulfonylchlorid $SOCl_2$ in einem chlorierten Lösungsmittel wie Dichlormethan zur Reaktion gebracht wird, um die chlorierte Verbindung (VIII) zu erhalten,

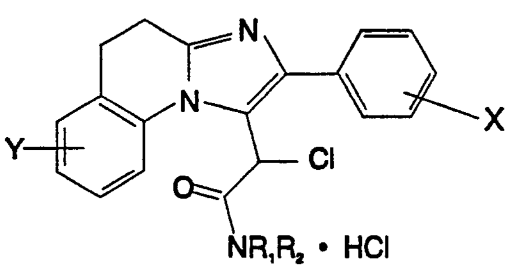

(VIII)

die mit Hilfe von Rongalite® in Methylenchlorid zur Verbindung (I) reduziert wird.

2. Verfahren nach Anspruch 1, dadurch gekennzeichnet, daß X ein Chloratom, eine Methylgruppe oder eine Methylthiogruppe in Stellung 4 bedeutet, Y ein Wasserstoffatom bedeutet und R1 und R2 einzeln jeweils unabhängig voneinander ein Wasserstoffatom oder eine Methylgruppe bedeuten.

**Claims** for the Contracting States: BE, CH, DE, FR, GB, IT, LI, LU, NL, SE

1. Imidazo[1,2-a]quinoline derivatives corresponding to the formula (I)

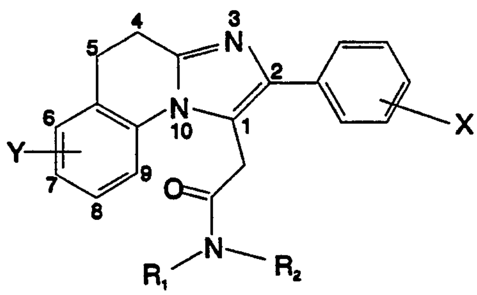

(I)

in which

X denotes hydrogen, a halogen or a $(C_{1-4})$alkyl, $(C_{1-4})$-alkoxy, $(C_{1-4})$alkylthio, methylsulphonyl, amino, $(C_{1-4})$-alkylamino, di$(C_{1-4})$alkylamino, nitro or trifluoroalkyl group,

Y denotes hydrogen, a halogen or a methyl group at position 6, 7 or 8, and

R1 and R2, taken separately, each denote hydrogen or a $(C_{1-6})$alkyl group, or alternatively

R1 and R2 together form a tetramethylene, pentamethylene, 3-methyl-3-azapentamethylene or 3-oxapentamethylene chain,

as well as their addition salts which are acceptable in pharmacology.

2. Derivatives according to Claim 1, characterized in that X denotes a chlorine atom, a methyl group or a methylthio group, at position 4, Y denotes a hydrogen atom, and R1 and R2 each denote, independently of one another, a hydrogen atom or a methyl group.

3. Process for preparing the compounds according to Claim 1, which process is characterized in that the quinoline of formula (II)

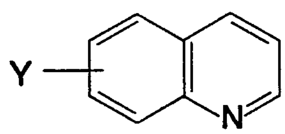

(II)

is first subjected to the action of an α-bromoacetophenone bearing the substituent X defined above, in the heated state in a solvent such as methylene chloride or 1,2-dichloroethane;
an ionic compound of formula (III)

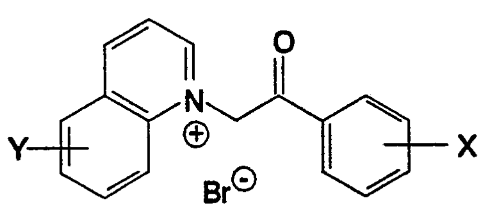

(III)

is obtained, which is cyclized in the presence of ammonium acetate, in an acidic organic solvent such as acetic or propionic acid, at a temperature of 90°C, to obtain the compound of formula (IV)

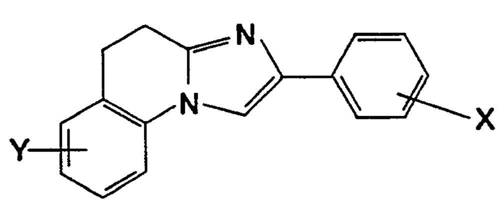

(IV)

the compound obtained (IV) is reacted with glyoxylic acid in a solvent such as acetic acid at 80°C; the α-hydroxy acid obtained (V)

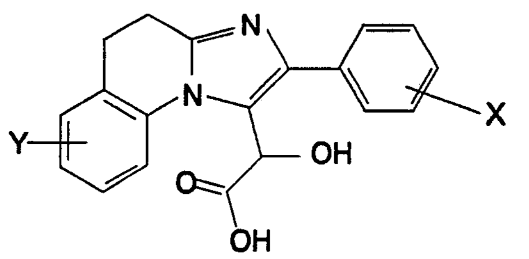

(V)

is acetylated using acetic anhydride in the presence of pyridine, and then converted to the α-acetoxyacetamide

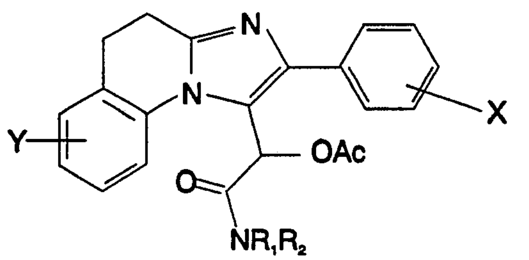

(VI)

via the imidazolide, which is prepared in situ; the compound (VI) is deacetylated to the α-hydroxyacetamide (VII)

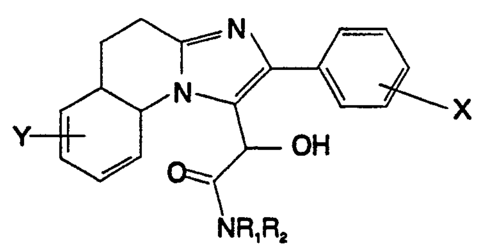

(VII)

by treatment with potassium carbonate in ethanol; the compound (VII) is reacted with sulphonyl chloride $SOCl_2$ in a chlorinated solvent such as dichloromethane, to obtain the chlorinated compound (VIII)

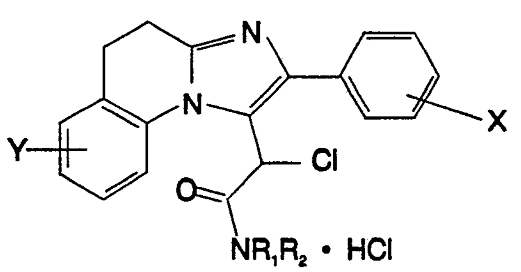

(VIII)

which is reduced using Rongalite® in methylene chloride to the compound (I).

4. Drug, characterized in that it consists of a compound according to either of Claims 1 and 2.

5. Pharmaceutical composition, characterized in that it contains a compound according to either of Claims 1 and 2, in combination with a suitable excipient.

**Claims** for the Contracting States: AT, ES, GR

1. Process for the preparation of imidazo[1,2-a]quinoline derivatives corresponding to the formula (I)

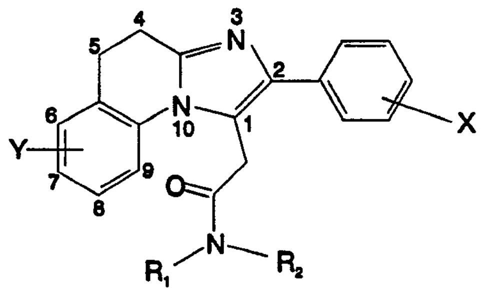

(I)

in which

X denotes hydrogen, a halogen or a $(C_{1-4})$alkyl, $(C_{1-4})$-alkoxy, $(C_{1-4})$alkylthio, methylsulphonyl, amino, $(C_{1-4})$-alkylamino, di$(C_{1-4})$alkylamino, nitro or trifluoroalkyl group,
Y denotes hydrogen, a halogen or a methyl group at position 6, 7 or 8, and
R1 and R2, taken separately, each denote hydrogen or a $(C_{1-6})$alkyl group, or alternatively
R1 and R2 together form a tetramethylene, pentamethylene, 3-methyl-3-azapentamethylene or 3-oxapentamethylene chain, which process is characterized in that the quinoline of formula (II)

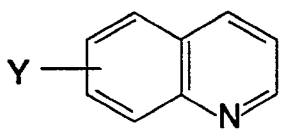

(II)

is first subjected to the action of an α-bromoacetophenone bearing the substituent X defined above, in the heated state in a solvent such as methylene chloride or 1,2-dichloroethane;
an ionic compound of formula (III)

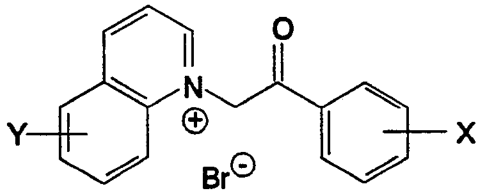

(III)

is obtained, which is cyclized in the presence of ammonium acetate, in an acidic organic solvent such as acetic or propionic acid, at a temperature of 90°C, to obtain the compound of formula (IV)

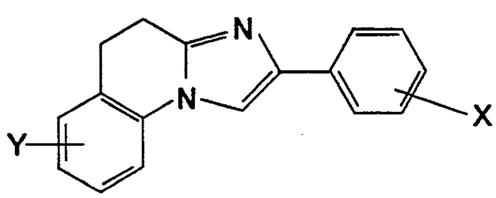

(IV)

the compound obtained (IV) is reacted with glyoxylic acid in a solvent such as acetic acid at 80°C;
the α-hydroxy acid obtained (V)

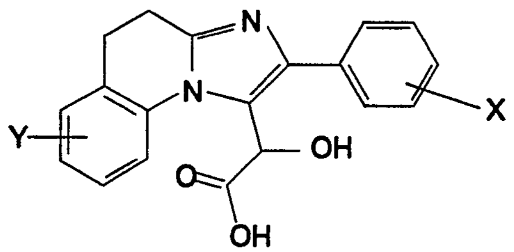

(V)

is acetylated using acetic anhydride in the presence of pyridine, and then converted to the α-acetoxyacetamide

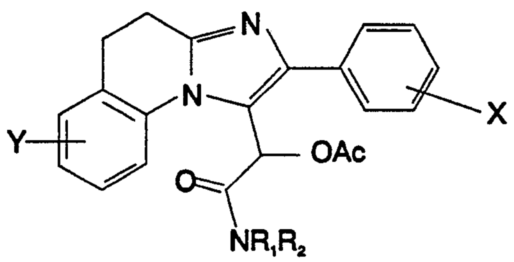

(VI)

via the imidazolide, which is prepared in situ; the compound (VI) is deacetylated to the α-hydroxyacetamide (VII)

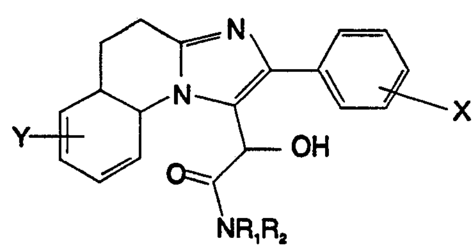

(VII)

by treatment with potassium carbonate in ethanol; the compound (VII) is reacted with sulphonyl chloride $SOCl_2$ in a chlorinated solvent such as dichloromethane, to obtain the chlorinated compound (VIII)

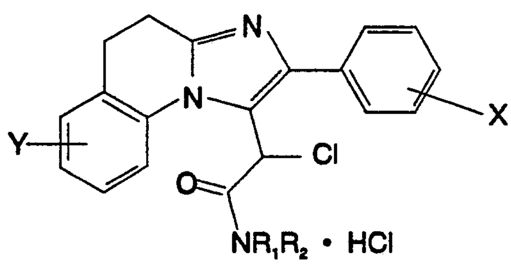

(VIII)

which is reduced using Rongalite® in methylene chloride to the compound (I).

2. Derivatives according to Claim 1, characterized in that X denotes a chlorine atom, a methyl group or a methylthio group, at position 4, Y denotes a hydrogen atom, and R1 and R2 each denote, independently of one another, a hydrogen atom or a methyl group.